# EUROPÄISCHE PATENTSCHRIFT

(11) **EP 2 034 309 B1**
(45) Veröffentlichungstag und Bekanntmachung des Hinweises auf die Patenterteilung: **18.11.2009**
(21) Anmeldenummer: 07114985.0
(22) Anmeldetag: 24.08.2007
(51) Int. Cl.: G01N 33/487, G01N 35/00

(54) **Analytisches System und Verfahren zu dessen Betrieb**
Analytic system and method for its operation
Système analytique et son procédé de fonctionnement

(43) Veröffentlichungstag der Anmeldung: 11.03.2009
(73) Patentinhaber: F.HOFFMANN-LA ROCHE AG, 4070 Basel (CH); Roche Diagnostics GmbH, 68305 Mannheim (DE)
(72) Erfinder: Schwöbel, Wolfgang, 68309 Mannheim (DE)
(74) Vertreter: Pfiz, Thomas

(56) Entgegenhaltungen:
- WO-A-2005/065828
- DE-A1- 10 360 786

## Beschreibung

Die Erfindung betrifft ein analytisches System und ein Arbeitsverfahren insbesondere für Blutzuckertests, bei dem eine Vielzahl von Testelementen in einer gegebenen Reihenfolge als Verbrauchseinheit in ein Gerät eingesetzt wird, und durch einen mit der Verbrauchseinheit koppelbaren Antrieb die Testelemente sukzessive an einer Messstelle bereitgestellt werden (siehe z.B. DE-A1-103 60 786).

Derartige Systeme dienen vor allem Diabetikern zur täglich mehrfach durchgeführten Blutzucker-Selbstkontrolle durch trägergebundene Schnelltests. Hierfür werden kompakte Handgeräte eingesetzt, die durch einen weitgehend automatischen Messablauf gewährleisten, dass auch von Laien die erforderlichen Schritte einfach und schnell vorgenommen werden können. Ein derartiges Analysegerät ist unter dem Handelsnamen ACCU-CHEK Compact am Markt erhältlich. Das bekannte Gerät arbeitet mit einem als Verbrauchsmittel auswechselbaren, in Segmente unterteilten Trommelmagazin, in welchem streifenförmige analytische Testelemente durch Trommelrotation der Reihe nach für eine Messung bereitstellbar sind. Bei einem Trommelwechsel wird jedoch nicht erkannt, ob es sich um ein neues oder bereits angebrauchtes Magazin handelt. Demgemäß wird von der Ausgangsposition jedes Trommelsegment angefahren, bis eine gebrauchsfähige Einheit bzw. ein vorhandener Teststreifen aufgefunden wird. Im Extremfall muss also der Anwender warten, bis alle Positionen durchlaufen und der letzte zur Verfügung stehende Test erreicht wird. Dies erfolgt auch, wenn das das Trommelfach nur für eine Gerätedemonstration oder Inspektionszwecke geöffnet und ohne Trommelwechsel wieder geschlossen wird.

Ausgehend hiervon liegt der Erfindung die Aufgabe zugrunde, die im Stand der Technik bekannten Systeme weiter zu verbessern und mit möglichst einfachen Mitteln eine weiter erhöhte Verbraucherfreundlichkeit und Verfahrenssicherheit zu gewährleisten.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, den Positionierablauf anhand einer Überprüfung der ersten Testposition zu steuern. Dementsprechend wird erfindungsgemäß vorgeschlagen, dass nach einer vorbestimmten Geräteaktuation der Antrieb zum Positionieren des in der Reihenfolge ersten Testelements an der Messstelle aktiviert wird und die Gebrauchsfähigkeit des Testelements ermittelt wird, und dass bei fehlender Gebrauchsfähigkeit des ersten Testelements das bei der Geräteaktuation zuletzt bereitgestellte unbenutzte Testelement wieder an der Messstelle positioniert wird. Weist die erste Magazinposition eine gebrauchsfähige Testeinheit auf, so geht das System davon aus, dass ein neues Magazin eingelegt wurde. Fehlt an der ersten Magazinposition eine gebrauchsfähige Einheit, so wird diejenige Magazinposition angefahren, die vor einer Geräteaktuation, die mit einem möglichen Magazinwechsel in Zusammenhang steht, zuletzt verwendet wurde. Dieser Ablauf lässt sich weitgehend ohne aufwändige Hilfsmittel und insbesondere ohne spezielle Magazinkodierung realisieren. Auf diese Weise wird also auch bei erneutem Einsatz des zuletzt benutzten Magazins ohne großen Zeitverlust ein Test für den Anwender möglich. Zudem kann die Energieversorgung des Systems geschont werden, was vor allem bei batteriebetriebenen Handgeräten wichtig ist.

Vorteilhafterweise wird eine Kennung des zuletzt bereitgestellten Testelements geräteseitig gespeichert, so dass das zuletzt bereitgestellte Testelement nach Maßgabe der Kennung wieder positioniert werden kann. Dies lässt sich beispielsweise dadurch realisieren, dass Positionsnummern für eine schrittweise Positionierung verwendet werden.

Möglich ist es auch, dass nach der Geräteaktuation der erforderliche Verfahrweg zum Positionieren des ersten Testelements erfasst wird, und dass bei fehlender Gebrauchsfähigkeit des ersten Testelements das zuletzt bereitgestellte Testelement entsprechend dem zuvor erfassten Verfahrweg wieder positioniert wird. Der Verfahrweg kann somit entsprechend der Wegdifferenz zwischen der bei der Geräteaktuation an der Messstelle befindlichen Testeinheit und der ersten Testeinheit bestimmt werden.

Die Ablaufsteuerung geht ohne zusätzliche Überprüfung davon aus, dass bei Gebrauchsfähigkeit des ersten Testelements eine neue Verbrauchseinheit eingewechselt wurde, so dass in diesem Fall die Testelemente in der gegebenen Reihenfolge für nachfolgende Tests eingesetzt werden.

Vorteilhaft ist es auch, wenn bei fehlender Gebrauchsfähigkeit des bei der Geräteaktuation zuletzt bereitgestellten Testelements eine durch die Reihenfolge der Testelemente in der Verbrauchseinheit gegebene Schrittfolge bis zur Gebrauchsfähigkeit eines Testelements abgefahren wird. Auf diese Weise ist auch der Fall abgedeckt, dass der Anwender eines von mehreren angebrauchten Magazinen willkürlich einsetzt.

Eine weitere Verbesserung der Benutzerfreundlichkeit ergibt sich dadurch, dass bei Gebrauchsfähigkeit eines Testelements die Testbereitschaft des Geräts für den Benutzer angezeigt wird.

Vorteilhafterweise wird systemseitig als Geräteaktuation eine mit einem möglichen Austausch der Verbrauchseinheit verbundene Handhabung durch einen Benutzer, beispielsweise das Einlegen einer Verbrauchseinheit, die Verriegelung einer Gerätekomponente oder das Schließen eines Gerätedeckels erkannt.

Eine besondere Ausführung sieht vor, dass die Testelemente vorzugsweise als Teststreifen in jeweils einer zugeordneten Kavität angeordnet sind, und dass die Gebrauchsfähigkeit durch eine Überprüfung des Vorhandenseins eines Testelements in der jeweiligen Kavität ermittelt wird. In diesem Zusammenhang ist es auch von Vorteil, wenn die Verbrauchseinheit durch ein drehbares Magazin, insbesondere ein Trommel- oder Scheibenmagazin gebildet wird, und wenn die Reihenfolge der Testeinheiten durch die Winkelposition von Segmenten des Magazins bestimmt wird. Entsprechend ist es auch denkbar, ein Stapelmagazin einzusetzen, bei welchem die Reihenfolge durch die Stapelposition definiert ist.

Um chargenspezifische Informationen verwerten zu können, ist es vorteilhaft, wenn die Verbrauchseinheit mit einem durch das Gerät abtastbaren Code versehen wird, und dass der Code zwischen der Geräteaktuation und dem Positionieren des ersten Testelements ausgelesen wird. Hierbei ist es günstig, als Code ein Barcode verwendet wird, und dass der Barcode unter Bewegung, insbesondere Drehung der Verbrauchseinheit berührungslos abgetastet wird.

Vorteilhafterweise werden die für Messungen benutzten Testelemente durch ein Zählwerk des Geräts gezählt und bis zum Austausch der Verbrauchseinheit im Zählwerk gespeichert, so dass eine vereinfachte Positionierung und Verbrauchserkennung möglich ist.

Gegenstand der Erfindung ist auch ein analytisches System für Patientenselbstkontrollen insbesondere für Blutzuckertests, umfassend ein Messgerät und eine darin einsetzbare Verbrauchseinheit, welche eine Vielzahl von analytischen Testelementen in einer gegebenen Reihenfolge enthält, wobei durch einen mit der Verbrauchseinheit koppelbaren Antrieb des Geräts die Testelemente sukzessive an einer Messstelle bereitstellbar sind. Zur Lösung der eingangs genannten Aufgabe wird hierbei vorgeschlagen, dass nach einer vorbestimmten Geräteaktuation eine Erfassungseinheit die Gebrauchsfähigkeit des in der Reihenfolge ersten Testelements ermittelt, und dass der Antrieb dazu ausgebildet ist, bei fehlender Gebrauchsfähigkeit des ersten Testelements das bei der Geräteaktuation zuletzt bereitgestellte Testelement an der Messstelle zu positionieren.

Im Folgenden wird die Erfindung anhand eines in der Zeichnung schematisch dargestellten Ausführungsbeispiels näher erläutert. Es zeigen
- Fig. 1: ein als Handgerät ausgebildetes analytisches System für Blutzuckertests mit einem Trommelmagazin für Teststreifen;
- Fig. 2: das als Verbrauchseinheit austauschbare Trommelmagazin mit einem ausgeschobenen Teststreifen in einer perspektivischen Darstellung;
- Fig. 3: ein Flussdiagramm des Positionierablaufs beim Einsetzen eines Trommelmagazins.

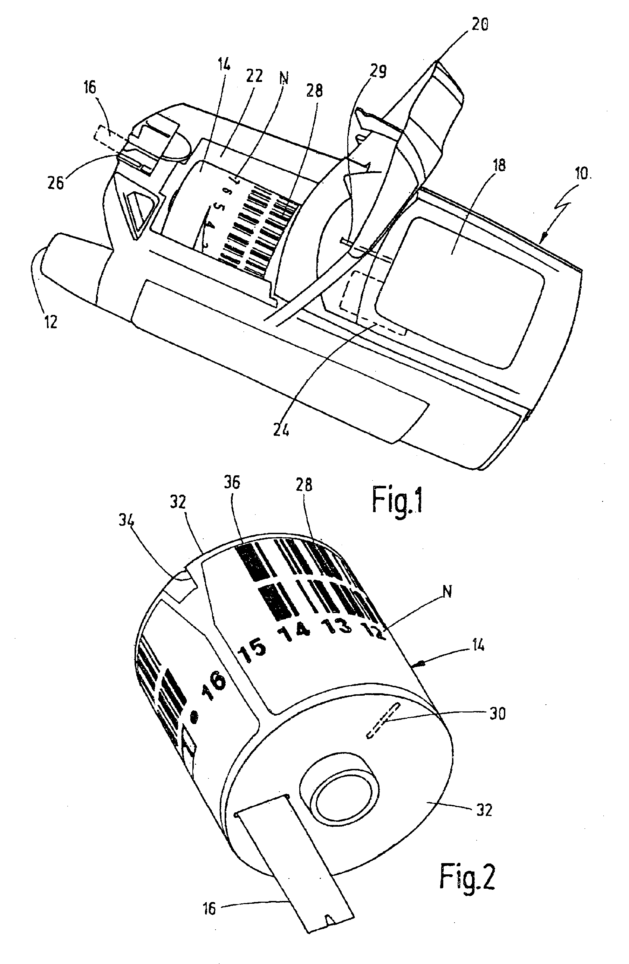

Das in Fig. 1 gezeigte Blutzuckermessgerät 10 ermöglicht Patientenselbstkontrollen des Glucosegehalts anhand einer mittels Stechhilfe 12 gewonnenen Blutprobe unter Einsatz einer als Verbrauchseinheit ausgebildeten Teststreifentrommel 14, welche eine Vielzahl von analytischen Teststreifen 16 enthält. Ein jeweils mit einem Blutstropfen beaufschlagter Teststreifen 16 lässt sich in einer nicht gezeigten geräteseitigen Messeinrichtung verarbeiten, wobei das Messergebnis auf einer Anzeige 18 vor Ort für den Benutzer angezeigt werden kann.

Wie in Fig. 1 veranschaulicht, ist die Teststreifentrommel 14 bei geöffnetem Gerätedeckel 20 in dem Trommelfach 22 zugänglich, so dass sie ggf. vom Benutzer inspiziert oder für eine Reinigung entnommen oder ausgetauscht werden kann. Nach dem Schließen des Deckels 20 wird die Teststreifentrommel 14 durch einen Drehantrieb 24 rotatorisch so positioniert, dass ein Teststreifen 16 an einer Messstelle 26 des Geräts 10 bereitgestellt werden kann. Gleichzeitig wird ein auf der Trommel aufgebrachter Barcode 28 gelesen, der das Messgerät über die Eigenschaften der Teststreifen 16 informiert.

Nach Auslösen einer Taste wird automatisch der in Zuordnung zu der Messstelle 26 positionierte Teststreifen 16 herausgeschoben. Dies erfolgt durch einen parallel zur Drehachse in die Trommel 14 eingreifenden Stößel 29. Nach der Messung wird der verbrauchte Teststreifen ausgestoßen, wobei das Messgerät 10 die verbrauchten Streifen 16 in einem Zählwerk zählt und die noch verfügbare Anzahl anzeigt. Weitere Gerätedetails ergeben sich aus der DE-A 103 60 786, auf die hier ausdrücklich Bezug genommen wird.

Die in Fig. 2 gezeigte Teststreifentrommel 14 besitzt in Umfangsrichtung verteilt angeordnete, axial durchgehende Streifenkammern 30, in denen jeweils ein Teststreifen 16 entsprechend den Positionskennungen N angeordnet ist. Stirnseitig ist die Trommel 14 durch Siegelfolien 32 verschlossen, die sich durch den Stößel 29 bzw. den Teststreifen 16 beim Ausschieben durchstoßen lassen. Eine Indexkerbe 34 kann für eine definierte Trommelpositionierung vorgesehen sein. Ebenso kann auch der erste Codebalken 36 des Barcodes 28 in einem vorbestimmten Winkelabstand zu der Position N1 des ersten Teststreifens 16 stehen.

Wie schon erwähnt, sollte nach einem Trommelwechsel bzw. nach dem Schließen des Gerätedeckels 20 sichergestellt werden, dass ein linear an die Messstelle 26 ausschiebbarer Teststreifen 16 zur Verfügung steht. Der entsprechende Positionierablauf sieht vor, dass zunächst die Kavität 30 des ersten Teststreifens 16 (Position N1) durch Trommelrotation zwischen Stößel 29 und Messstelle 26 positioniert wird. Das Gerät 10 geht folglich in jedem Fall zuerst davon aus, dass eine unbenutzte Teststreifentrommel 14 eingelegt wurde. Sodann wird das Vorhandensein des ersten Teststreifens 16 überprüft. Bei Nichtvorhandensein des ersten Teststreifens wird der vor dem Trommelwechsel zuletzt bereitgestellte Teststreifen (Position Nx) durch geeignete Trommelrotation wieder bereitgestellt. In diesem Fall wird also angenommen, dass die zuletzt verwendete und dabei nur angebrauchte Teststreifentrommel 14 wieder eingesetzt wurde.

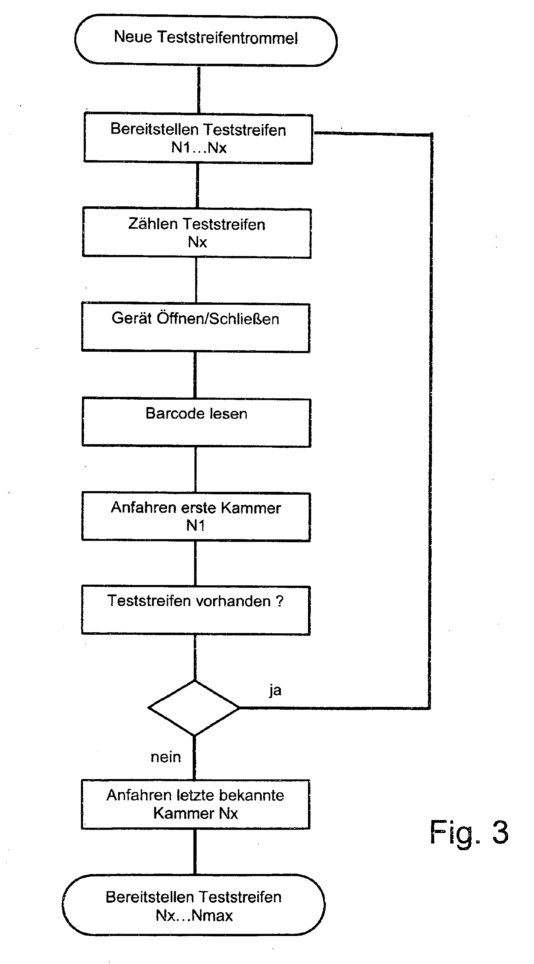

Die Ablaufsteuerung im Einzelnen ergibt sich aus dem Flussdiagramm gemäß Fig. 3. Das Gerät 10 stellt nach dem Einsatz einer neuen Teststreifentrommel durch Trommeldrehung mittels des Antriebs 24 sukzessive die Teststreifen 16 zum Ausschub an der Messstelle 26 bereit. Die Positionsnummer Nx des zuletzt bereitgestellten Streifens wird in einem Zählwerk gespeichert.

Nach Öffnen und Schließen des Deckels 20 wird zunächst der Barcode ausgelesen, wofür eine Trommelumdrehung gegenüber einem gerätefesten Codeleser erforderlich ist. Dabei kann ein in dem Barcode enthaltener Chargencode erfasst und für die Auswertung der Tests berücksichtigt werden.

In einem nächsten Schritt wird die erste Kavität bzw. Kammer 30 angefahren, d.h. die Trommelposition N1 an der Messstelle 26 gegenüber dem Stößel 29 positioniert. Dies kann ausgehend von der Indexkerbe 34 oder dem ersten Codebalken 36 durch einen vorbestimmten Drehweg erfolgen. Die Überprüfung auf das Vorhandensein eines Teststreifens kann durch einen Tastvorschub des Stößels 29 erfolgen, wobei die erforderliche Antriebskraft erfasst wird. Bei noch nicht durchstoßener Siegelfolie 32 kann somit aufgrund des erhöhten Widerstands auf einen noch vorhandenen Teststreifen geschlossen werden. Es versteht sich, dass eine Überprüfung auch auf andere Weise, beispielsweise optisch erfolgen kann. Das Zählwerk wird dann zurückgesetzt und die Teststreifen werden durch schrittweises Weiterschalten entsprechend ihrer Reihenfolge sukzessive bereitgestellt.

Befindet sich jedoch an der ersten Position N1 kein Teststreifen, wird die gespeicherte Position Nx des vor der Geräteaktuation, d.h. dem Öffnen und Schließen zuletzt bereitgestellten Teststreifens angefahren. Dies lässt sich durch eine entsprechend der Positionsdifferenz bestimmte Trommelrotation auf einfache Weise bewerkstelligen. Möglich ist es auch, dass der Rotationsweg nach der Geräteaktuation bis zum Erreichen der ersten Position erfasst wird, und dass die Trommel bei Nichtvorhandensein des ersten Teststreifens um einen entsprechenden Weg zurück positioniert wird.

Bei Erreichen der letzten bekannten Kammer (Nx) wird erneut das Vorhandensein eines Teststreifens 16 geprüft. Ist der Teststreifen vorhanden, werden die folgenden Kammern 30 erst für nachfolgende Tests bis zum Erreichen der maximalen Zahl Nmax angefahren. Denkbar ist es jedoch auch, dass der Benutzer eine zweite angebrauchte Teststreifentrommel gleicher Charge eingelegt hat, so dass an der Position Nx gegebenenfalls kein Teststreifen vorhanden ist. In diesem Fall werden die Folgepositionen Nx bis Nmax schrittweise abgefahren, bis eventuell ein Teststreifen aufgefunden wird.

Der vorstehend beschriebene Ablauf ist nicht auf den Einsatz von Teststreifentrommeln beschränkt. Grundsätzlich kommen auch andere Wechselmagazine, beispielsweise Scheiben- bzw. Stapelmagazine oder Bandkassetten und andere analytische Testelemente, beispielsweise Testbandfelder oder Chips in Betracht. Denkbar ist die Anwendung bei beliebigen (integrierten) Disposables als Testelementen, die eine Stech- und/oder Nachweisfunktion bei der Analyse von Körperflüssigkeiten erfüllen.

## Patentansprüche

1. Verfahren zum Betrieb eines analytischen Systems insbesondere für Blutzuckertests, bei dem
a) eine Vielzahl von Testelementen (16) in einer gegebenen Reihenfolge als Verbrauchseinheit (14) in ein Gerät (10) eingesetzt wird, und
b) durch einen mit der Verbrauchseinheit (14) koppelbaren Antrieb (24) die Testelemente (16) sukzessive an einer Messstelle (26) bereitgestellt werden,
c) wobei nach einer vorbestimmten Geräteaktuation der Antrieb (24) zum Positionieren des in der Reihenfolge ersten Testelements (16) an der Messstelle (26) aktiviert wird und die Gebrauchsfähigkeit des Testelements (16) ermittelt wird, und
d) bei fehlender Gebrauchsfähigkeit des ersten Testelements (16) das bei der Geräteaktuation zuletzt bereitgestellte Testelement (16) wieder an der Messstelle (26) positioniert wird.

2. Verfahren nach Anspruch 1, **dadurch gekennzeichnet, dass** eine Kennung (N) des zuletzt bereitgestellten Testelements (16) geräteseitig gespeichert wird, und dass das zuletzt bereitgestellte Testelement (16) nach Maßgabe der Kennung (N) wieder positioniert wird.

3. Verfahren nach Anspruch 1 oder 2, **dadurch gekennzeichnet, dass** nach der Geräteaktuation der erforderliche Verfahrweg zum Positionieren des ersten Testelements (16) erfasst wird, und dass bei fehlender Gebrauchsfähigkeit des ersten Testelements (16) das zuletzt bereitgestellte Testelement (16) entsprechend dem Verfahrweg wieder positioniert wird.

4. Verfahren nach einem der Ansprüche 1 bis 3, **dadurch gekennzeichnet, dass** bei Gebrauchsfähigkeit des ersten Testelements (16) die Testelemente in der gegebenen Reihenfolge in der Verbrauchseinheit (14) für nachfolgende Tests eingesetzt werden.

5. Verfahren nach einem der Ansprüche 1 bis 4, **dadurch gekennzeichnet, dass** bei fehlender Gebrauchsfähigkeit des bei der Geräteaktuation zuletzt bereitgestellten Testelements (16) eine durch die Reihenfolge der Testelemente in der Verbrauchseinheit (14) gegebene Schrittfolge bis zur Gebrauchsfähigkeit eines Testelements (16) abgefahren wird.

6. Verfahren nach einem der Ansprüche 1 bis 5, **dadurch gekennzeichnet, dass** bei Gebrauchsfähigkeit eines Testelements (16) die Testbereitschaft des Geräts (10) für einen Benutzer angezeigt wird.

7. Verfahren nach einem der Ansprüche 1 bis 6, **dadurch gekennzeichnet, dass** als Geräteaktuation eine mit einem möglichen Austausch der Verbrauchseinheit (14) verbundene Handhabung durch einen Benutzer, beispielsweise das Einlegen einer Verbrauchseinheit (14), die Verriegelung einer Gerätekomponente oder das Schließen eines Gerätedeckels (20) erkannt wird.

8. Verfahren nach einem der Ansprüche 1 bis 7, **dadurch gekennzeichnet, dass** die Testelemente (16) vorzugsweise als Teststreifen in jeweils einer zugeordneten Kavität (30) angeordnet sind, und dass die Gebrauchsfähigkeit durch eine Überprüfung des Vorhandenseins eines Testelements (16) in der jeweiligen Kavität (30) ermittelt wird.

9. Verfahren nach einem der Ansprüche 1 bis 8, **dadurch gekennzeichnet, dass** die Verbrauchseinheit (14) durch ein drehbares Magazin, insbesondere ein Trommel- oder Scheibenmagazin gebildet wird, und dass die Reihenfolge der Testeinheiten (16) durch die Winkelposition von Segmenten des Magazins bestimmt wird.

10. Verfahren nach einem der Ansprüche 1 bis 9, **dadurch gekennzeichnet, dass** die Verbrauchseinheit (14) mit einem durch das Gerät (10) abtastbaren Code (28) versehen wird, und dass der Code (28) zwischen der Geräteaktuation und dem Positionieren des ersten Testelements (16) ausgelesen wird.

11. Verfahren nach Anspruch 10, **dadurch gekennzeichnet, dass** als Code (28) ein Barcode verwendet wird, und dass der Barcode unter Bewegung, insbesondere Drehung der Verbrauchseinheit (14) berührungslos abgetastet wird.

12. Verfahren nach einem der Ansprüche 1 bis 11, **dadurch gekennzeichnet, dass** die für Messungen benutzten Testelemente durch ein Zählwerk des Geräts (10) gezählt werden, und dass die Anzahl der benutzten Testelemente bis zum Austausch der Verbrauchseinheit (14) im Zählwerk gespeichert werden.

13. Analytisches System für Patientenselbstkontrollen insbesondere für Blutzuckertests, umfassend ein Messgerät und eine darin einsetzbare Verbrauchseinheit (14), welche eine Vielzahl von analytischen Testelementen (16) in einer gegebenen Reihenfolge enthält, wobei durch einen mit der Verbrauchseinheit (14) koppelbaren Antrieb (24) des Geräts (10) die Testelemente sukzessive an einer Messstelle (26) bereitstellbar sind, **dadurch gekennzeichnet, dass** nach einer vorbestimmten Geräteaktuation eine Erfassungseinheit die Gebrauchsfähigkeit des in der Reihenfolge ersten Testelements (16) ermittelt, und dass der Antrieb (24) dazu ausgebildet ist, bei fehlender Gebrauchsfähigkeit des ersten Testelements (16) das bei der Geräteaktuation zuletzt bereitgestellte Testelement (16) an der Messstelle (26) zu positionieren.

## Claims

1. Process for operating an analytical system, in particular for blood sugar tests in which
a) a plurality of test elements (16) is inserted in a given order into a device (10) as a disposable unit (14), and
b) the test elements (16) are successively provided at a measuring position (26) by a drive (24) that can be coupled with the disposable unit (14),
c) wherein after a predetermined device actuation, the drive (24) is activated to position the test element (16) that is first in order at the measuring position (26) and it is determined whether the test element (16) can be used, and
d) if it is not possible to use the first test element (16), the test element (16) that was provided last when the device was actuated is again positioned at the measuring position (26).

2. Process according to claim 1, **characterized in that** an identifier (N) of the test element (16) that was provided last is stored in the device and that the test element (16) that was provided last is positioned again according to the identifier (N).

3. Process according to claim 1 or 2, **characterized in that** after the device has been actuated, the required travel path to position the first test element (16) is recorded and if the first test element (16) cannot be used, the test element (16) provided last is positioned again according to the travel path.

4. Process according to one of the claims 1 to 3, **characterized in that** if the first test element (16) is usable the test elements are used for subsequent tests in the given order in the disposable unit (14).

5. Process according to one of the claims 1 to 4, **characterized in that** if the test element (16) provided last upon the device actuation is unusable, a sequence of steps determined by the order of the test elements in the disposable unit (14) is executed until a test element (16) is usable.

6. Process according to one of the claims 1 to 5, **characterized in that** if a test element (16) is usable, the test readiness of the device (10) is indicated to the user.

7. Process according to one of the claims 1 to 6, **characterized in that** an operation by a user associated with a possible replacement of the disposable unit (14) such as the insertion of a disposable unit (14), the locking of a device component or the closing of a device cover (20) is identified as the device actuation.

8. Process according to one of the claims 1 to 7, **characterized in that** the test elements (16) are arranged preferably as test strips each in one allocated cavity (30) and that the usability is determined by checking the presence of a test element (16) in the respective cavity (30).

9. Process according to one of the claims 1 to 8, **characterized in that** the disposable unit (14) is formed by a rotatable magazine and in particular a drum or disk magazine and that the order of test units (16) is determined by the angular position of segments of the magazine.

10. Process according to one of the claims 1 to 9, **characterized in that** the disposable unit (14) is provided with a code (28) that can be scanned by the device (10) and that the code (28) is read between the device actuation and the positioning of the first test element (16).

11. Process according to claim 10, **characterized in that** a barcode is used as the code (28) and that the barcode is scanned without contact while the disposable unit (14) is moved and in particular rotated.

12. Process according to one of the claims 1 to 11, **characterized in that** the test elements used for measurements are counted by a counter of the device (10) and that the number of used test elements is stored in the counter until the disposable unit (14) is replaced.

13. Analytical system for patient self-monitoring especially for blood sugar tests comprising a measuring device and a disposable unit (14) that can be inserted therein which contains a plurality of analytical test elements (16) in a given order where the test elements can be successively provided at a measuring position (26) by a drive (24) of the device (10) which can be coupled with the disposable unit (14), **characterized in that** after a predetermined device actuation, a detection unit determines the usability of the test element (16) that is first in order and that the drive (24) is designed such that if the first test element (16) is unusable, the test element (16) that was provided last when the device was actuated is positioned at the measuring position (26).

## Revendications

1. Procédé pour le fonctionnement d'un système analytique en particulier pour des tests de glycémie, dans lequel
a) une pluralité d'éléments de test (16) est insérée dans un appareil (10) dans un ordre de succession donné comme unité de consommation (14), et
b) les éléments de test (16) sont mis à disposition de façon successive en un point de mesure (26) par un entraînement (24) pouvant être couplé avec l'unité de consommation (14),
c) l'entraînement (24) étant activé, après un actionnement d'appareil prédéfini, pour le positionnement du premier élément de test (16) dans l'ordre de succession sur le point de mesure (26) et l'aptitude à l'utilisation de l'élément de test (16) étant déterminée, et
d) en cas d'absence d'aptitude à l'utilisation du premier élément de test (16), le dernier élément de test (16) mis à disposition lors de l'actionnement de l'appareil est positionné à nouveau au point de mesure (26).

2. Procédé selon la revendication 1, **caractérisé en ce qu'**un code (N) du dernier élément de test (16) mis à disposition est stocké côté appareil, et **en ce que** le dernier élément de test (16) mis à disposition est positionné à nouveau en fonction du code (N).

3. Procédé selon la revendication 1 ou 2, **caractérisé en ce que**, après l'actionnement de l'appareil, la course de déplacement nécessaire pour le positionnement du premier élément de test (16) est enregistrée, et **en ce que**, en cas d'absence d'aptitude à l'utilisation du premier élément de test (16), le dernier élément de test (16) mis à disposition est positionné à nouveau en fonction de la course de déplacement.

4. Procédé selon l'une quelconque des revendications 1 à 3, **caractérisé en ce que**, en cas d'aptitude à l'utilisation du premier élément de test (16), les éléments de test sont utilisés dans l'ordre de succession donné dans l'unité de consommation (14) pour des tests consécutifs.

5. Procédé selon l'une quelconque des revendications 1 à 4, **caractérisé en ce que**, en cas d'absence d'aptitude à l'utilisation du dernier élément de test (16) mis à disposition lors de l'actionnement de l'appareil, une succession d'étapes donnée par l'ordre de succession des éléments de test (16) dans l'unité consommation (14) est mise en route jusqu'à l'aptitude à l'utilisation d'un élément de test (16).

6. Procédé selon l'une quelconque des revendications 1 à 5, **caractérisé en ce que**, en cas d'aptitude à l'utilisation d'un élément de test (16), la disponibilité pour le test de l'appareil (10) pour un utilisateur est indiquée.

7. Procédé selon l'une quelconque des revendications 1 à 6, **caractérisé en ce qu'**une manipulation liée à un remplacement possible de l'unité de consommation (14) par un utilisateur, par exemple la mise en place d'une unité de consommation (14), le verrouillage d'un composant d'appareil ou la fermeture d'un couvercle d'appareil (20) est reconnue comme actionnement d'appareil.

8. Procédé selon l'une quelconque des revendications 1 à 7, **caractérisé en ce que** les éléments de test (16) sont disposés de préférence sous forme de bande de test dans respectivement une cavité (30) associée, et **en ce que** l'aptitude à l'utilisation est déterminée par un contrôle de la présence d'un élément de test (16) dans la cavité (30) respective.

9. Procédé selon l'une quelconque des revendications 1 à 8, **caractérisé en ce que** l'unité de consommation (14) est formée par un chargeur rotatif, en particulier un chargeur à tambour ou à disque et **en ce que** l'ordre de succession des éléments de test (16) est déterminé par la position angulaire de segments du chargeur.

10. Procédé selon l'une quelconque des revendications 1 à 9, **caractérisé en ce que** l'unité de consommation (14) est dotée d'un code (28) pouvant être balayé par l'appareil (10), et **en ce que** le code (28) est lu entre l'actionnement de l'appareil et le positionnement du premier élément de test (16).

11. Procédé selon la revendication 10, **caractérisé en ce qu'**un code-barres est utilisé comme code (28) et **en ce que** le code-barres est balayé sans contact par déplacement, en particulier la rotation de l'unité de consommation (14).

12. Procédé selon l'une quelconque des revendications 1 à 11, **caractérisé en ce que** les éléments de test utilisés pour des mesures sont comptés par un compteur de l'appareil (10), **en ce que** le nombre des éléments de test utilisés est stocké jusqu'au remplacement de l'unité de consommation (14) dans le compteur.

13. Système analytique pour autocontrôles de patients en particulier pour des tests de glycémie, comprenant un appareil de mesure et une unité de consommation (14) pouvant être insérée à l'intérieur, qui contient une pluralité d'éléments de test (16) analytiques dans un ordre de succession donné, les éléments de test pouvant être mis à disposition de façon successive en un point de mesure (26) par un entraînement (24), pouvant être couplé avec l'unité de consommation (14), de l'appareil (10), **caractérisé en ce que**, après un actionnement prédéfini de l'appareil, une unité d'enregistrement détermine l'aptitude à l'utilisation du premier élément de test (16) dans l'ordre de succession, et **en ce que** l'entraînement (24) est conçu pour positionner le dernier élément de test (16) mis à disposition lors de l'actionnement de l'appareil sur le point de mesure (26) en cas d'absence d'aptitude à l'utilisation du premier élément de test (16).
